# EUROPEAN PATENT SPECIFICATION

(11) **EP 2 344 892 B1**
(45) Date of publication and mention of the grant of the patent: **21.05.2014**
(21) Application number: 09740779.5
(22) Date of filing: 30.09.2009
(51) Int. Cl.: G01N 33/94

(54) **Gucuronidated acetaminophen as a marker of hepatic disorders**
Glucuronidiertes Acetaminophen als Marker für Lebererkrankungen
Acétaminophène glucuronidé en tant que marqueur de troubles hépatiques

(30) Priority: 01.10.2008 US 194835 P
(43) Date of publication of application: 20.07.2011
(73) Proprietor: The Arizona Board Of Regents On Behalf Of The University Of Arizona, Tucson, AZ 85721-0158 (US)
(72) Inventor: CHERRINGTON, Nathan, Oro Valley,AZ 85755 (US)
(74) Representative: Lee, Nicholas John
(86) International application number: PCT/US2009/058913
(87) International publication number: WO 2010/039754

(56) References cited:
- EL-AZAB G ET AL: "Acetaminophen plasma level after oral administration in liver cirrhotic patients suffering from schistosomal infection" INTERNATIONAL JOURNAL OF CLINICAL PHARMACOLOGY AND THERAPEUTICS, vol. 34, no. 7, 1996, pages 299-303, XP8117148 ISSN: 0946-1965
- FORREST J A H ET AL: "PARACETAMOL METABOLISM IN CHRONIC LIVER DISEASE" EUROPEAN JOURNAL OF CLINICAL PHARMACOLOGY, SPRINGER VERLAG, DE, vol. 15, no. 6, 1 November 1979 (1979-11-01), pages 427-431, XP008117151 ISSN: 0031-6970 [retrieved on 2004-12-12]
- GMYREK D: "Results of administration of large oral dose of paracetamol to children with infectious hepatitis" DTSCHXSESUNDH.WE 1971, vol. 26, no. 11, 1971, pages 503-508, XP008117173
- CORCORAN G B ET AL: "OBESITY AS A RISK FACTOR IN DRUG-INDUCED ORGAN INJURY INCREASED LIVER AND KIDNEY DAMAGE BY ACETAMINOPHEN IN THE OBESE OVERFED RAT" JOURNAL OF PHARMACOLOGY AND EXPERIMENTAL THERAPEUTICS, vol. 241, no. 3, 1987, pages 921-927, XP8117295 ISSN: 0022-3565
- LICKTEIG ANDREW J ET AL: "Efflux transporter expression and acetaminophen metabolite excretion are altered in rodent models of nonalcoholic fatty liver disease" DRUG METABOLISM AND DISPOSITION, vol. 35, no. 10, October 2007 (2007-10), pages 1970-1978, XP002563826 ISSN: 0090-9556 cited in the application
- SHINODA SHIGEO ET AL: "Pharmacokinetics/pharmacodynamics of acetaminophen analgesia in Japanese patients with chronic pain" BIOLOGICAL & PHARMACEUTICAL BULLETIN, vol. 30, no. 1, January 2007 (2007-01), pages 157-161, XP002563827 ISSN: 0918-6158
- LIANG M Z ET AL: "Pharmacokinetics of Mycophenolic Acid and Its Glucuronide After a Single and Multiple Oral Dose of Mycophenolate Mofetil in Chinese Renal Transplantation Recipients" TRANSPLANTATION PROCEEDINGS, ORLANDO, FL, US, vol. 38, no. 7, 1 September 2006 (2006-09-01), pages 2044-2047, XP025008414 ISSN: 0041-1345 [retrieved on 2006-09-01]

## Description

### Background of the Invention

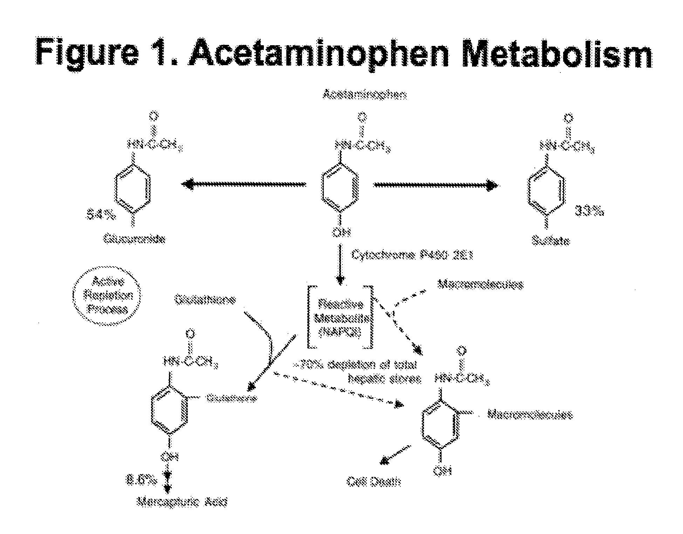

Acetaminophen (APAP) is the most commonly used non-opioid analgesic and the standard antipyretic and analgesic agent against which most other similar products are compared. The metabolism of APAP, the vast majority of which occurs in liver, is well defined **(****Figure 1****).** At least one-half of an administered dose is conjugated with glucuronic acid (GLUC) and one-third with sulfate (SULF). The CYP-450-dependent mixed-function oxidase enzyme pathway, primarily via CYP2E1, metabolizes approximately 5-9%. This results in the formation of a toxic reactive intermediate metabolite (NAPQI), which is subsequently conjugated with glutathione to form nontoxic cysteine and mercapturic acid conjugates. Less than 5% of a recommended acetaminophen dose is excreted unchanged by the kidneys. Acetaminophen undergoes first-order elimination from the body and has a short plasma half-life (2.0-2.4 hours).

APAP provides a fine example of the integral role played by efflux transporters in drug metabolite excretion from the liver. All APAP metabolites require efflux transport in order to be excreted from the liver, and each can be detected in both bile and urine. In vivo disposition studies and in vitro functional transport experiments indicate that the ABC transporters ABCC2, ABCC3, ABCC4 and ABCG2 each have the ability to transport a variety of unconjugated and conjugated drugs, including APAP metabolites. ABCC2 and ABCG2 are localized to the canalicular (apical) membrane of hepatocytes from which they excrete their substrates into the bile canaliculi. Accordingly, in a healthy liver, biliary excretion of the SULF, GLUC and GSH conjugates of APAP is predominantly mediated by ABCC2, while ABCG2 appears also to contribute to excretion of APAP-SULF conjugates. ABCC3 and ABCC4 are expressed at the sinusoidal (basolateral) membrane ofhepatocytes and cholangiocytes from which they expel their substrates into the blood. Sinusoidal excretion of the APAP-GLUC metabolite from hepatocytes is predominantly mediated by ABCC3, while ABCC4 appears to mediate excretion of APAP-SULF metabolites. Recent studies indicate that ABCC3 and ABCC4 have an equal role in the efflux of APAP-SULF.

A recent study (Drug. Metabolism and Disposition, 35:1970-1978 (2007)) has shown that in a rat model of non-alcoholic steatohepatitis (NASH), generated by feeding rats a methionine-, choline-deficient (MCD) diet for eight weeks, liver efflux transporters ABCC3 and ABCC4 increase in protein expression. Administration to these MCD rats of large doses of APAP (1 mmol/kg) (equivalent to a 10 gram dose in a 150 pound human, or a 17 gram dose in a 250 pound human) resulted in a shift from biliary to plasma excretion of APAP-GLUC relative to control. However, given that there are no methionine-, choline-deficient humans, and the large dosages of APAP administered to the rat model, it has been speculated that the MCD rat is not a good model for human NASH or for studies of human liver drug metabolism. It is generally accepted that no animal model accurately recapitulates human NASH. Numerous biochemical differences make any direct comparison potentially fraught with artifacts. For example, the MCD model is lacking the insulin resistance that is often a component of NASH (Rinella ME and Green RM, J Hepatol. 2004 Jan;40(1):47-51.). Furthermore, whereas steatosis in humans occurs in a typical western diet high in fat resulting in an increased net flux of lipids through the liver, the MCD model causes steatosis and steatohepatitis by a biochemical impairment of lipid metabolism. Thus, direct comparison between the MCD model and humans is inappropriate. This is especially a concern for the MCD diet, as the effects of this unnatural diet versus effects of liver damage on their variables cannot be delineated.
El-Azab G et al, International Journal Of Clinical Pharmacology And Therapeutics, vol. 34, no. 7, 1996, pages 299-303 describes a study in which the plasma level of acetaminophen and its glucuronide and sulfate metabolites was determined in liver-cirrhotic patients suffering from schistosomal infection and compared with that in corresponding healthy subject groups. Plasma concentration of acetaminophen glucuronide was greatly decreased in the liver-cirrhotic patients relative to the control.
In Forrest J A H et al, European Journal Of Clinical Pharmacology, Springer Verlag, De, vol. 15, no. 6, 1 November 1979 (1979-11-01), pages 427-431, the plasma concentrations and urinary excretion of paracetamol and its glucuronide, sulphate, cysteine and mercapturic acid conjugates were measured in eight normal subjects, eight patients with mild liver disease and seven patients with severe liver disease following an oral dose of 1.5 g of paracetamol. The mean ratios of plasma concentrations of unchanged paracetamol to paracetamol glucuronide and sulphate were significantly greater in patients with severe liver disease than the normal subjects.
Gmyrek D et al, TSCHXSESUNDH.WE 1971, vol. 26, no. 11, 1971, pages 503-508 discloses an investigation into the usability of a paracetemol (NAPAP) loading test as a liver function test in acute hepatitis. It was found that the ratio of free NAPAP to NAPAP glucuronide was increased in urine samples from patients with hepatitis. The ratio was reduced following treatment of the disease in hospital.
Corcoran G B Et Al, Journal Of Pharmacology And Experimental Therapeutics, Vol. 241, No. 3, 1987, Pages 921-927 describes the use of acetaminophen as a prototype drug to examine the influence of obesity on the hepatotoxic and nephrotoxic potential of metabolically activated drugs. The analysis is carried out in an obese rat model. The conclusion is that obese rats form more glucuronide conjugate than controls.
In Shinoda Shigeo et al, Biological & Pharmaceutical Bulletin, Vol. 30, No. 1, January 2007 (2007-01), Pages 157-161, the pharmacokinetics and pharmacodynamics of APAP in healthy volunteers and patients with chronic pain was studied. Plasma APAP and APAP metabolite concentrations were measured in the volunteers and plasma APAP concentrations and pain scores were measured in the patients with chronic pain. It was suggested that the pharmacokinetic and pharmacodynamic parameters measured could be used to determine an effective APAP dosage regimen for patients with chronic pain.

### Summary of the Invention

In a first aspect, the present invention provides a method for diagnosing non-alcoholic steatohepatitis (NASH) or hepatic fibrosis in a human, comprising
(a) determining an amount of acetaminophen (APAP)-glucuronide in a plasma sample and/or a urine sample obtained from a human subject after the human subject was administered acetaminophen (APAP), wherein the human subject is at risk of non-alcoholic steatohepatitis (NASH) or hepatic fibrosis;
(b) comparing the amount of APAP-glucuronide in one or both of the plasma sample and the urine sample to a control; and
(c) identifying the human subject as having non-alcoholic steatohepatitis (NASH) or hepatic fibrosis if the amount of APAP-glucuronide in one or both of the plasma sample and the urine sample is elevated relative to the control.

In a second aspect, the present invention provides a method for identifying a human subject at risk of an adverse drug reaction, comprising
(a) determining an amount of acetaminophen (APAP)-glucuronide in a plasma sample and/or a urine sample obtained from a human subject after the human subject was administered acetaminophen (APAP), wherein the human subject is in need of treatment with a therapeutic drug selected from the group consisting of doxorubicin, morphine, cisplatin, ectoposide, methotrexate, glucuronidated conjugated drugs, glutathione-conjugated drugs, and sulfate-conjugated drugs;
(b) comparing the amount of APAP-glucuronide in one or both of the plasma sample and the urine sample to a control; and
(c) identifying the human subject as at risk of an adverse drug reaction if the amount of APAP-glucuronide in one or both of the plasma sample and the urine sample is elevated relative to the control.

In a third aspect, the present invention provides a method for determining an appropriate dosage of a therapeutic drug selected from the group consisting of doxorubicin, morphine, cisplatin, ectoposide, methotrexate, glucuronidated conjugated drugs, glutathione-conjugated drugs, and sulfate-conjugated drugs for a human subject, comprising
(a) determining an amount of acetaminophen (APAP)-glucuronide in a plasma sample and/or a urine sample obtained from a human subject after the human subject was administered acetaminophen (APAP), wherein the human subject is in need of treatment with a therapeutic drug selected from the group consisting of doxorubicin, morphine, cisplatin, ectoposide, methotrexate, glucuronidated conjugated drugs, glutathione-conjugated drugs, and sulfate-conjugated drugs;
(b) comparing the amount of APAP-glucuronide in one or both of the plasma sample and the urine sample to a control; and
(c) determining that the human subject should receive a non-standard dosage of the drug if the amount of APAP-glucuronide in one or both of the plasma sample and the urine sample is elevated relative to the control.

In a fourth aspect, the present invention provides machine readable storage media, comprising a set of instructions for causing a metabolite measuring device to carry out the determining, comparing, and identifying steps of any embodiment of the methods of the first, second, and third aspects.

Embodiments of each of these aspects of the invention are provided herein.

### Brief Description of the Figures

**Figure 1** is a flow-chart showing the metabolism of acetaminophen.
**Figure 2** provides autoradiographs showing the expression of hepatic ABCC3 and ABCC4 in humans with NAFLD.
**Figure 3A-B** show ABCC2 protein localized to the very crisp edges of the canalicular membrane in livers from normal healthy patients (A), but clearly blurred into membrane vesicles away from the canaliculus in NASH livers (B).
**Figure 4** shows the concentration of APAP and its major metabolites in plasma over 4 hours.
**Figure 5** is a graph showing the concentration of APAP and APAP metabolites in the urine of normal healthy volunteers, patients with steatosis, and patients with steatohepatitis.
**Figure 6A-C** are graphs of plasma APAP-Gluc analyses for NASH (A), hepatic inflammation (B), and hepatic fibrosis (C).

### Detailed Description of the Invention

Within this application, unless otherwise stated, the techniques utilized may be found in any of several well-known references such as: Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), "Guide to Protein Purification" in Methods in Enzymology (M.P. Deutshcer, ed., (1990) Academic Press, Inc.); PCR Protocols: A Guide to Methods and Applications (Innis, et al. 1990. Academic Press, San Diego, CA), Culture of Animal Cells: A Manual of Basic Technique, 2nd Ed. (R.I. Freshney. 1987. Liss, Inc. New York, NY), Gene Transfer and Expression Protocols, pp. 109-128, ed. E.J. Murray, The Humana Press Inc., Clifton, N.J.), and the Ambion 1998 Catalog (Ambion, Austin, TX).

As used herein, the singular forms "a", "an" and "the" include plural referents unless the context clearly dictates otherwise. "And" as used herein is interchangeably used with "or" unless expressly stated otherwise.

In a first aspect, the present invention provides a method for diagnosing non-alcoholic steatohepatitis (NASH) or hepatic fibrosis in a human, comprising
(a) determining an amount of APAP-glucuronide in a plasma sample and/or a urine sample obtained from a human subject after the human subject was administered acetaminophen (APAP), wherein the human subject is at risk of non-alcoholic steatohepatitis (NASH) or hepatic fibrosis;
(b) comparing the amount of APAP-glucuronide in one or both of the plasma sample and the urine sample to a control; and
(c) identifying the human subject as having non-alcoholic steatohepatitis (NASH) or hepatic fibrosis if the amount of APAP-glucuronide in one or both of the plasma sample and the urine sample is elevated relative to the control.

The inventor has demonstrated that the methods of the invention provide a non-invasive blood test or urine test to identify human patients with a variety of hepatic disorders.

The human subject may be adolescent or adult. Plasma and/or urine samples may be obtained from the human subject by standard techniques well known to those of skill in the art.

As used herein, the term "hepatic disorder" includes any liver disease in humans that exhibits a modification of acetaminophen metabolism as demonstrated herein, including but not limited to hepatic fibrosis, hepatitis, non-alcoholic steatohepatitis (NASH), alcoholic steatohepatitis, toxin induced steatohepatitis, primary sclerosing cholangitis, cirrhosis, and sepsis.

As used herein, "acetaminophen" (APAP) is the widely known pain and fever reducer, and formulations thereof, of the structure shown in Figure 1. Any suitable dosage of APAP can have been administered to the human subject. While more than one dose of APAP may have been administered to the human subject, the indicated maximal dose of 1000 mg is preferred to minimize the risk of toxicity while still facilitating analysis of metabolite formation. In one preferred embodiment of all aspects and embodiments of the invention, between 250 mg and 1000 mg total dosage of APAP has been administered to the human subject. In one preferred embodiment, the human subjects has not taken any APAP for at least 24 hours before the test. In one preferred embodiment of all aspects and embodiments of the invention, between 250 mg and 1000 mg total dosage of APAP has been administered to the human subject. Any suitable formulation of APAP may have been administered, including but not limited to liquid oral dosage forms and solid oral dosage forms.

As used herein, "APAP-glucuronide" refers to one specific metabolite of APAP, with the structure shown in **Figure 1****.**

As used herein, a "control" is any means for normalizing the amount of APAP-glucuronide being measured in the human subject with a healthy liver. In one preferred embodiment, the control comprises pre-defined APAP-glucuronide levels from a normal individual or population (i.e.: known not to be suffering from the hepatic disorder of interest).

As used herein, an "elevated" amount of APAP-glucuronide relative to control can be any increase above control, and preferably is a statistically significant increase (e.g. p ≤ 0.05). In various preferred embodiments, the elevated level of APAP-glucuronide is at a 1.2-fold, 1.4-fold, 1.5-fold, 2-fold, 3-fold, 4-fold, 5-fold, or greater difference in the amount of APAP-glucuronide in the blood or urine sample relative to appropriate control.

Plasma and/or urine samples obtained at any suitable time after APAP administration for detection of APAP-glucuronide can be used in the methods of all aspects and embodiments of the invention. In one preferred embodiment of all aspects and embodiments of the invention, the amount of APAP-glucuronide is measured in a plasma sample, and the measurement is made between 1 minute and 180 minutes after APAP administration; more preferably between 1 minute and 120 minutes after APAP administration, and even more preferably between 1 minute and 60 minutes after APAP administration. In various further preferred embodiments, the measurement is made between 10 minutes and 180 minutes; 30 minutes and 180 minutes; 45 minutes and 180 minutes; 60 minutes and 180 minutes; 10 minutes and 120 minutes; 30 minutes and 120 minutes; 45 minutes and 120 minutes; 10 minutes and 60 minutes; 30 minutes and 60 minutes; and 45 minutes and 60 minutes. In another preferred embodiment of all aspects and embodiments of the invention, the amount of APAP-glucuronide is measured in a urine sample, and wherein a measurement is made between 120 minutes and 480 minutes after APAP administration; more preferably between 120 minutes and 360 minutes after APAP administration; and even more preferably between 120 minutes and 300 minutes after APAP administration or between 120 minutes and 240 minutes after APAP administration. In various further preferred embodiments, the measurement is made between 180 minutes and 480 minutes; 180 minutes and 360 minutes; 180 minutes and 300 minutes; 180 minutes and 240 minutes; 240 minutes and 480 minutes; 240 minutes and 360 minutes; 240 minutes and 300 minutes; 300 minutes and 480 minutes; 300 minutes and 360 minutes; 360 minutes and 480 minutes. In various further preferred embodiments, 1, 2, 3, 4, or more measurements may be made, to, for example, assess increases in APAP-glucuronide compared to control at one or more time points. The methods may further comprise detection and/or measurement of other analytes in the plasma and/or urine, including but not limited to APAP, APAP-sulfate, APAP-NAC and APAP-CG/CYS.

Any suitable processing steps may be carried out to prepare the plasma and/or urine sample for APAP-glucuronide measurement. In one exemplary embodiment, proteins are precipitated from the plasma and/or urine samples using standard techniques, and centrifuged; the resulting supernatant is then used for the measurement step of the methods of the invention.

Measuring an amount of APAP-glucuronide in one or both of the plasma and the urine sample can be carried out by any suitable technique for analyte measurement, including but not limited to chromatography (such as high pressure liquid chromatography (HPLC), gas chromatography (GC), GC-mass spectrometry (GC-MS), thin-layer chromatography (TLC), etc.), nuclear magnetic resonance (NMR), spectroscopy electrochemical techniques, capillary electrophoresis, and immunological techniques.

In a further preferred embodiment of any of the above embodiments, the determining, comparing, and identifying steps are automated; in this embodiment, these steps can be carried out using an automated instrument, such a device for carrying out any of the techniques above. Such devices would incorporate machine readable storage media comprising instructions for causing the device to carry out the determining, comparing, and identifying steps.

Plasma and/or urine samples obtained from a human test subject may be stored prior to measuring APAP-GLUC; suitable storage conditions are known to those of skill in the art. For example, samples may be frozen at suitable temperatures for the desired length of storage; in one embodiment, the samples are stable for up to one year at -80°C.

In the first aspect, the human subject is at risk of non-alcoholic steatohepatitis (NASH). Nonalcoholic Fatty Liver Disease (NAFLD) comprises a spectrum of pathologic lesions that ranges from hepatic steatosis to a form of fatty liver hepatitis known as non-alcoholic steatohepatitis (NASH), the most severe form of NAFLD. Histologic features independently associated with the diagnosis of NASH in human biopsies include hepatic steatosis, hepatocyte ballooning, lobular inflammation, Mallory's hyaline and perisinusoidal fibrosis. Moreover, NASH has the potential to advance to cirrhosis requiring liver transplant.

As disclosed herein, the inventor has discovered that in human NASH patients, the ABCC2 transporter is not correctly localized at the canalicular membrane, but is instead localized into membrane vesicles away from the canaliculus in NASH livers. This aberrant localization of the ABCC2 transporter was not identified in the rat MCD model described in Lickteig et al. (2007). The inventor has also discovered that expression of hepatic ABCC3 and ABCC4 is up-regulated in human NASH patients. While not being bound by any mechanism of action, the inventor believes that the altered hepatic trafficking of ABCC2 to membrane vesicles alters the disposition of APAP-GLUC to favor plasma retention via ABCC3 and/or ABCC4 (and then excretion into urine), rather than biliary clearance through ABCC2.

The methods of this embodiment can serve to stratify subjects suffering from NAFLD, and distinguish those suffering from NASH from those with hepatic steatosis. Current methods for diagnosing NASH rely on liver biopsy. Thus, non-invasive methods for diagnosing NASH are of clinical value, and permit more rapid onset of treatment for NASH, including but not limited to aggressive weight-reduction programs and therapeutic drug treatment.

In a preferred embodiment, the human subject at risk of NASH suffers from one or more of non-alcoholic fatty liver disease, steatosis metabolic syndrome, obesity, dyslipidemia, insulin resistance, cardiovascular disease, and diabetes.

In the first aspect of the invention, the human subject is alternatively at risk of hepatic fibrosis. Hepatic fibrosis is overly exuberant wound healing in which excessive connective tissue builds up in the liver. The extracellular matrix is either overproduced, degraded deficiently, or both. Fibrosis itself causes no symptoms but can lead to portal hypertension (the scarring distorts blood flow through the liver) or cirrhosis (the failure to properly replace destroyed liver cells results in liver dysfunction). Current methods for diagnosing liver fibrosis rely on liver biopsy. Thus, non-invasive methods for diagnosing liver fibrosis are of clinical value. In a preferred embodiment, the human subject at risk of liver fibrosis suffers from, or previously suffered from, one or more conditions predisposing to liver fibrosis selected from the group consisting of α₁-antitrypsin deficiency, Wilson's disease, fructosemia, galactosemia, Type III, IV, VI, IX, and X glycogen storage diseases, hemochromatosis, Gaucher's disease, Zellweger syndrome, tyrosinemia, bacterial infection, viral infection (such as by hepatitis B virus (HBV) or hepatitis C virus (HCV), parasitic infection, Budd-Chiari syndrome, alcoholism, drug addiction, heart failure, hepatic veno-occlusive disease, biliary obstruction, and portal vein thrombosis.

As disclosed in more detail below, the inventor has discovered that human subjects with hepatic fibrosis have an elevated amount of APAP-GLUC in plasma and urine than human subjects without hepatic fibrosis. The methods of the invention permit more rapid onset of treatment for hepatic fibrosis, including but not limited to removing the basis of the liver injury, for example by antiviral therapy for eliminating HBC or HCV; abstaining from alcohol in alcoholic liver disease; removing heavy metals such as iron or copper in hemochromatosis and Wilson's disease, respectively; or decompressing bile ducts in biliary obstruction.

Liver fibrosis is generally histologically stratified as mild (minimal scarring around blood vessels), moderate (scarring extending out from the liver blood vessels), or severe (scarring forming bridges between blood vessels). A human subject can be identified as having severe liver fibrosis if the amount of APAP-glucuronide in one or both of a plasma and a urine sample is elevated relative to the control. The human subject may be suffering from NASH.

Disclosed herein is a method for diagnosing a human subject at risk of hepatitis, an inflammation of the liver characterized by diffuse or patchy necrosis. Major causes are specific hepatitis viruses, alcohol, and drugs. Thus, the human subject with hepatitis may suffer from, or have previously suffered from one or more disorders selected from the group consisting of alcoholism, drug addiction, bacterial infection, viral infection (such as hepatitis A, B, C, D, and/or E), fungal infection, protozoan infection, helminth infection, spirochete infection, sarcoidosis, ulcerative colitis, and Crohn's disease.

As disclosed in more detail below, the inventor has discovered that human subjects with hepatic inflammation (hepatitis) have an elevated amount of APAP-GLUC in plasma and urine than human subjects without hepatic fibrosis. Liver inflammation is generally histologically stratified as mild (less than 2 foci of inflammation), moderate (2-4 foci of inflammation), or severe (more than 4 foci of inflammation). A human subject may be identified as having moderate or severe hepatic inflammation if the amount of APAP-glucuronide in one or both of a plasma and a urine sample is elevated relative to the control. A human subject may be identified as having severe hepatic inflammation if the amount of APAP-glucuronide in one or both of a plasma and a urine sample is elevated relative to the control. The human subject may be suffering from NASH and/or hepatic fibrosis.

In a second aspect, the present invention provides a method for identifying a human subject at risk of an adverse drug reaction, comprising
(a) determining an amount of APAP-glucuronide in a plasma sample and/or a urine sample obtained from a human subject after the human subject was administered acetaminophen (APAP), wherein the human subject is in need of treatment with a therapeutic drug selected from the group consisting of doxorubicin, morphine, cisplatin, ectoposide, methotrexate, glucuronidated conjugated drugs, glutathione-conjugated drugs, and sulfate-conjugated drugs;
(b) comparing the amount of APAP-glucuronide in one or both of the plasma sample and the urine sample to a control; and
(c) identifying the human subject as at risk of an adverse drug reaction if the amount of APAP-glucuronide in one or both of the plasma sample and the urine sample is elevated relative to the control.

In a third aspect, the present invention provides a method for determining an appropriate dosage of a therapeutic drug selected from the group consisting of doxorubicin, morphine, cisplatin, ectoposide, methotrexate, glucuronidated conjugated drugs, glutathione-conjugated drugs, and sulfate-conjugated drugs for a human subject, comprising
(a) determining an amount of APAP-glucuronide in a plasma sample and/or a urine sample obtained from a human subject after the human subject was administered acetaminophen (APAP), wherein the human subject is in need of treatment with a therapeutic drug selected from the group consisting of doxorubicin, morphine, cisplatin, ectoposide, methotrexate, glucuronidated conjugated drugs, glutathione-conjugated drugs, and sulfate-conjugated drugs;
(b) comparing the amount of APAP-glucuronide in one or both of the plasma sample and the urine sample to a control; and
(c) determining that the human subject should receive a non-standard dosage of the drug if the amount of APAP-glucuronide in one or both of the plasma sample and the urine sample is elevated relative to the control.

All common terms between the first, second, and third aspects of the invention share the same definition; all embodiments of the first aspect are applicable to the second and third aspects of the invention unless the context clearly dictates otherwise.

As disclosed herein, the inventor has discovered that in human NASH patients, the ABCC2 transporter is not correctly localized at the canalicular membrane, but is instead localized into membrane vesicles away from the canaliculus in NASH livers. This aberrant localization of the ABCC2 transporter was not identified in the rat MCD model described in Lickteig et al. (2007). The inventor has also discovered that expression of hepatic ABCC3 and ABCC4 is up-regulated in human NASH patients. While not being bound by any mechanism of action, the inventor believes that the altered hepatic trafficking of ABCC2 to membrane vesicles alters the disposition of APAP-GLUC to favor plasma retention via ABCC3 and/or ABCC4 (and then excretion into urine), rather than biliary clearance through ABCC2. The proportional shift in elimination of APAP metabolites from bile to plasma/urine thus indicates alterations in efflux transporter expression and/or cellular localization that can affect the route of drug elimination, and thus provides methods to identify those human subjects that are at risk of an adverse drug reaction due to alterations in efflux transporter expression and/or localization.

As used herein, "at risk of an adverse drug reaction" means that the human subject is more likely to experience one or more adverse reactions associated with the drug to be received. Such possible adverse reactions include any such adverse reactions, both those listed in the product literature for any given drug, as well as idiosyncratic adverse reactions.

As used herein, a "non-standard dosage of the drug" means that an attending physician should alter a dosage of drug to be administered to the human subject to take into account an increased risk of an adverse drug reaction.

In various preferred embodiments of the second and third aspects of the invention, the human subject in need of therapeutic drug treatment is at risk or suffers from NASH, hepatic fibrosis, and/or hepatic inflammation. Human subjects at risk of each of these disorders are disclosed in detail in the first aspect of the invention. In another preferred embodiment, the human subject suffers from any disorder leading to an alteration in ABCC2, ABCC3, and/or ABCC4 transporter expression and/or localization. In one preferred embodiment, the disorder leads to aberrant localization of the ABCC2 transporter.

The therapeutic drug is selected from doxorubicin, cisplatin, ectoposide, methotrexate, morphine, ezeitimibe, glucuronidated conjugated drugs, glutathione-conjugated drugs, and sulfate-conjugated drugs, i.e. whose metabolite(s) are transported from the liver via the ABCC2, ABCC3, and/or ABCC4 transporters.

Any suitable dosage of APAP can have been administered to the human subject. While more than one dose of APAP may have been administered to the human subject, in a preferred embodiment a single dosage has been administered to facilitate analysis of metabolite formation. In one preferred embodiment, the human subjects have not taken any APAP for at least 24 hours before the test. In a further preferred embodiment of all aspects and embodiments of the invention, between 250 mg and 1000 mg total dosage of APAP has been administered to the human subject. Any suitable formulation of APAP may have been administered, including but not limited to liquid oral dosage forms and solid oral dosage forms.

Plasma and/or urine samples obtained at any suitable time after APAP administration for detection of APAP-glucuronide can be used in the methods of all aspects and embodiments of the invention. In one preferred embodiment of all aspects and embodiments of the invention, the amount of APAP-glucuronide is measured in a plasma sample, and the measurement is made between 1 minute and 240 minutes after APAP administration; more preferably between 1 minute and 180 minutes after APAP administration, and even more preferably between 1 minute and 120 or between 1 minute and 60 minutes after APAP administration. In another preferred embodiment of all aspects and embodiments of the invention, the amount of APAP-glucuronide is measured in a urine sample, and wherein a measurement is made between 120 minutes and 480 minutes after APAP administration; more preferably between 120 minutes and 360 minutes after APAP administration; and even more preferably between 120 minutes and 300 minutes or between 120 minutes and 240 minutes after APAP administration.

Any suitable processing steps may be carried out to prepare the plasma and/or urine sample for APAP-glucuronide measurement. In one exemplary embodiment, proteins are precipitated from the plasma and/or urine samples using standard techniques, and centrifuged; the resulting supernatant is then used for the measurement step of the methods of the invention.

Measuring an amount of APAP-glucuronide in one or both of the plasma and the urine sample can be carried out by any suitable technique for analyte measurement, including but not limited to chromatography (such as high pressure liquid chromatography (HPLC), gas chromatography (GC), GC-mass spectrometry (GC-MS), thin-layer chromatography (TLC), etc.), nuclear magnetic resonance (NMR), spectroscopy electrochemical techniques, capillary electrophoresis, and immunological techniques.

As used herein, a "control" is any means for normalizing the amount of APAP-glucuronide being measured in the human subject with a healthy liver. In one preferred embodiment, the control comprises pre-defined APAP-glucuronide levels from a normal individual or population (ie: known not to be suffering from the hepatic disorder of interest).

As used herein, an "elevated" amount of APAP-glucuronide relative to control can be any increase above control, and preferably is a statistically significant increase (e.g. p ≤ 0.05). In various preferred embodiments, the elevated level of APAP-glucuronide is at a 1.2-fold, 1.4-fold, 1.5-fold, 2-fold, 3-fold, 4-fold, 5-fold, or greater difference in the amount of APAP-glucuronide in the blood or urine sample relative to appropriate control.

In a further preferred embodiment of any of the above embodiments, the determining, comparing, and identifying steps are automated; in this embodiment, these steps can be carried out using an automated instrument, such a device for carrying out any of the techniques above. Such devices would incorporate machine readable storage media comprising instructions for causing the device to carry out the determining, comparing, and identifying steps.

In a fourth aspect, the present invention provides a machine readable storage media, comprising a set of instructions for causing a metabolite measuring device to carry out the determining, comparing, and identifying steps of any embodiment of methods of the first, second, and third aspects of the invention. As used herein the term "computer readable medium" includes magnetic disks, optical disks, organic memory, and any other volatile (e.g., Random Access Memory ("RAM")) or non-volatile (e.g., Read-Only Memory ("ROM")) mass storage system readable by the CPU. The computer readable medium includes cooperating or interconnected computer readable medium, which exist exclusively on the processing system or be distributed among multiple interconnected processing systems that may be local or remote to the processing system. As used herein, "metabolite measuring device" means a device capable of carrying out the metabolite measurements to carry out the invention, including, but not limited to chromatography devices (such as high pressure liquid chromatography (HPLC) devices, gas chromatography (GC) devices, GC-mass spectrometry (GC-MS) devices, thin-layer chromatography (TLC) devices, etc.), nuclear magnetic resonance (NMR) devices, spectroscopy devices, electrochemical devices, immunological detection devices, and capillary electrophoresis devices.

### Reference Example 1: Increased hepatic efflux transporter expression in humans diagnosed with NASH

Studies were carried out to determine whether patients suffering from NAFLD show altered metabolism and disposition of acetaminophen. Human liver samples from patients with normal liver, steatosis, NASH with fatty liver and end stage NASH (cryptogenic cirrhosis) were obtained from the NIH funded Liver Tissue Procurement and Distribution System (LTPADS).

Immunoblot Protein Analysis. Whole cell lysates (20µg/well) were separated by SDS-PAGE on 7.5% gels and transferred to PVDF membranes overnight at 30 constant milliamps. All membranes were blocked for one hour at room temperature in 5% non-fat dry milk made in phosphate-buffered saline with Tween 20 (PBST). Membranes were then incubated in primary antibody solution (1:2000 dilution in PBST-milk) consisting of either ABCC3, ABCC4 (Abcam, Cambridge, MA) monoclonal, or GAPDH (Santa Cruz Biotechnology, Santa Cruz, CA) polyclonal antibodies overnight at 4°C. Following overnight incubation, membranes were incubated in HRP-conjugated secondary antibody (Santa Cruz Biotechnology, Santa Cruz, CA) solutions for one hour at room temperature. Antibody binding was detected using the ECL Advance Western Blotting Detection Kit (GE Healthcare Life Sciences, Piscataway, NJ) per the manufacturer's protocol.

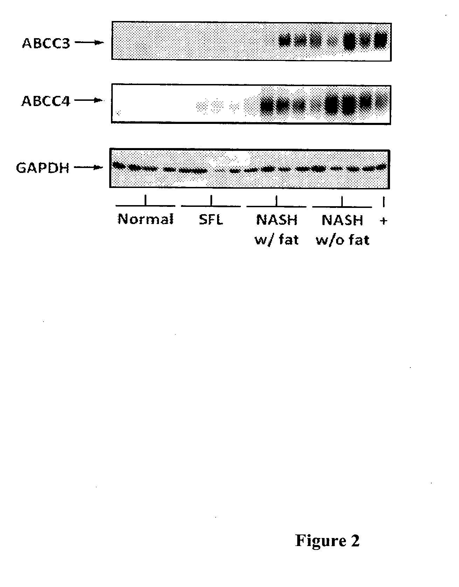

**Figure 2** shows the expression of hepatic ABCC3 and ABCC4 in humans with NAFLD. There was little change in ABCC3 and ABCC4 expression in livers with steatosis when compared to normal liver; however, there is a dramatic increase in protein expression in human livers with either stage of NASH. Whereas the expression of these transporters in human was previously thought to have large inter-individual variation, it appears that the variation may be in large part due to disease state.

### Reference Example 2: Cellular localization of ABCC2 is altered in human livers diagnosed with NASH.

Altered cellular trafficking as a means of regulating the function of ABCC2 has been well documented in various conditions such as drug induced toxicities and pregnancy. A requirement for ABCC2 activity is the proper localization at the canalicular membrane. Moving the ABCC2 transporter away from the canalicular membrane into membrane vesicles may be an evolutionary advantage that allows the cell to quickly regulate transport activity without having to wait for the relatively slow process of protein degradation/de novo synthesis.

Immunohistochemical Staining. Briefly, tissue sections from formalin-fixed paraffin-embedded human liver samples were deparaffinized in xylene and re-hydrated in ethanol, followed by antigen retrieval in citrate buffer (pH 6.0). Endogenous peroxidase activity was blocked with 0.3% (v/v) H2O2 in methanol for 20 minutes. All slides were placed in Shandon Sequenza™ Slide Racks (Thermo-Scientific, Waltham, MA) for remaining reagent incubations. Background staining was blocked by incubating all samples in Background Sniper™ (Biocare Medical, Concord, CA) for 10 minutes. Samples were then incubated in a mouse monocolonal MRP2 antibody overnight at 4°C. The following day, samples were incubated in Mouse Probe and HRP Polymer (Biocare Medical, Concord, CA) for 15 minutes each to detect bound antibody. Color development was performed by incubation in Betazoid™ DAB (Biocare Medical, Concord, CA) for 3 minutes followed by quenching in deionized water. All samples were counterstained with freshly filtered hematoxilin solution (Sigma-Aldrich, St. Louis, MO). All slides were imaged with a Nikon Eclipse™ E4000 microscope and a Sony Exwave™ DXC-390 camera.

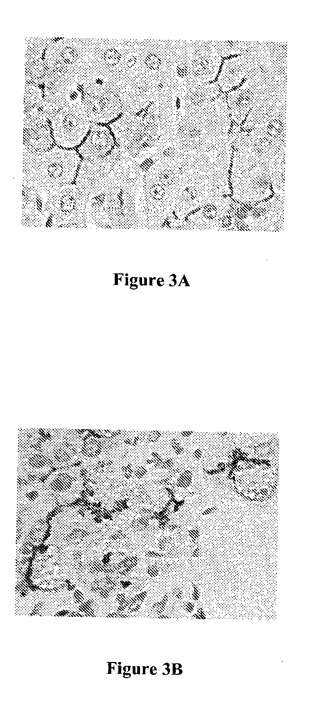

As seen in **Figure 3****,** ABCC2 protein is localized to the very crisp edges of the canalicular membrane in livers from normal healthy patients, but is clearly blurred into membrane vesicles away from the canaliculus in NASH livers. This mechanism of trafficking ABCC2 away from its site of activity allows the hepatocyte to effectively regulate and conserve important endogenous components during cellular stress, while maintaining protein expression. Altered cellular trafficking of ABCC2 to membrane vesicles alters the disposition of APAP-GLUC to favor plasma retention rather than biliary clearance.

### Example 3: Acetaminophen-Glucuronide levels are higher in the plasma and urine of pediatric patients with NASH than in patients with steatosis.

A study was conducted in pediatric patients whose disease status had been biopsy verified. Twenty-four pediatric patients (12-18yrs) were desirable for the preliminary study due to the presumed lack of confounding factors (e.g., alcoholic consumption, illicit drug use) and the increasing prevalence of childhood obesity. Conversely, pediatric patients are limited in the amount of real liver damage that can take place simply due to age, where pediatric patients are incapable of developing ballooning degeneration and are limited to fat deposition, inflammation, and fibrosis.

High Performance Liquid Chromatography Analysis. APAP and its metabolites in plasma, and urine samples were quantified by high performance liquid chromatography analysis based on previously described methods (Howie et al., 1977a;Chen et al., 2000;Slitt et al., 2003d). APAP and its metabolites were resolved using a Zorbmax™ SB-C 18 reverse-phase 4.6-mm x 25-cm column with a Phenomenex™ Security Guard Column Guard and eluted using a mobile phase composed of 12.5% HPLC-grade methanol, 1% acetic acid, and 86.5% water, run isocratically at a flow rate of 1.2 mL/minute. The elution of metabolites was monitored at a wavelength of 254 nm. Retention times of APAP and its metabolites were determined by comparison with that of authentic standards. Since this HPLC method does not separate the cysteinyl glycine and cysteine conjugates of APAP, they were quantified together as APAP-CG/CYS. Samples were analyzed using a Beckman System Gold™ HPLC system (Beckman Coulter, Inc., Fullerton, CA) equipped with a 128-nm solvent module and a 166-nm detector. Quantitation was based on integrated peak areas. The concentrations of APAP and its metabolites were calculated using an APAP standard curve since the molar extinction coefficients of APAP and its conjugated metabolites are approximately the same (Howie et al., 1977b). To precipitate proteins in plasma, and urine, samples were diluted 1:2, 1:2, and 1:3, respectively, with ice-cold methanol and centrifuged at 4,000 x g for 30 minutes at 4°C. The resulting supernatants were collected and diluted in mobile phase 1:3 (urine) or 1:2 (plasma) prior to HPLC analysis.

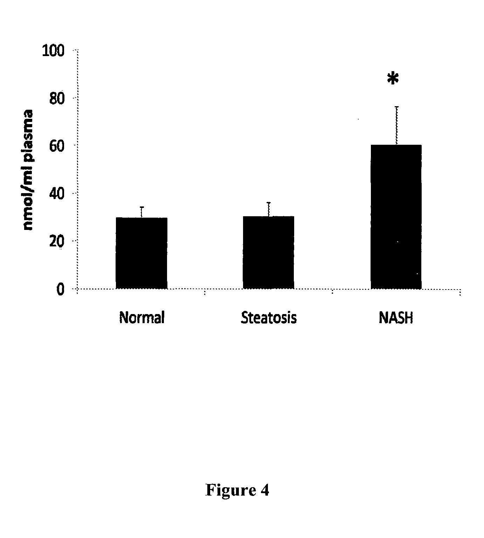

**Figure 4** shows the concentration of APAP and its major metabolites in plasma 1 hour after APAP administration. Mean and median data are shown in Table 1.

**Table 1**

| | Mean (nmol/ml) | Median (nmol/ml) |
|---|---|---|
| Normal 1h | 29.9 | 29.0 |
| Normal 2h | 54.3 | 48.9 |
| Normal 3h | 46.7 | 39.2 |
| Steatosis 1h | 30.5 | 32.6 |
| Steatosis 2h | 44.3 | 41.1 |
| Steatosis 4h | 35.5 | 35.3 |
| NASH 1h | 60.7 | 75.9 |
| NASH 2h | 72.7 | 66.4 |
| NASH 4h | 68.3 | 62.6 |

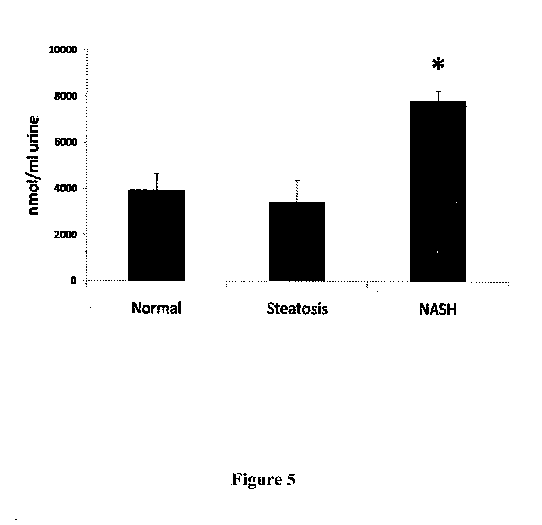

The concentration of APAP- GLUC in plasma of patients with steatohepatitis was significantly higher than in patients with simple steatosis at all time points measured. Additionally, Figure 5 shows the concentration of APAP and APAP metabolites in the urine of normal healthy volunteers, patients with steatosis, and patients with steatohepatitis. Differences in the levels of APAP- GLUC are not significantly different between patients with NASH and other groups until four hours due to the time constraints of urine production and bladder emptying, as well as the requirement of hepatic metabolism and subsequent distribution to the kidney. These data clearly indicate the potential to develop a non-invasive blood test or urine test to delineate patients with NASH from those simply with steatosis.

### Example 4. Acetaminophen-Glucuronide levels are higher in the plasma of pediatric patients with hepatic fibrosis than in patients without hepatic fibrosis

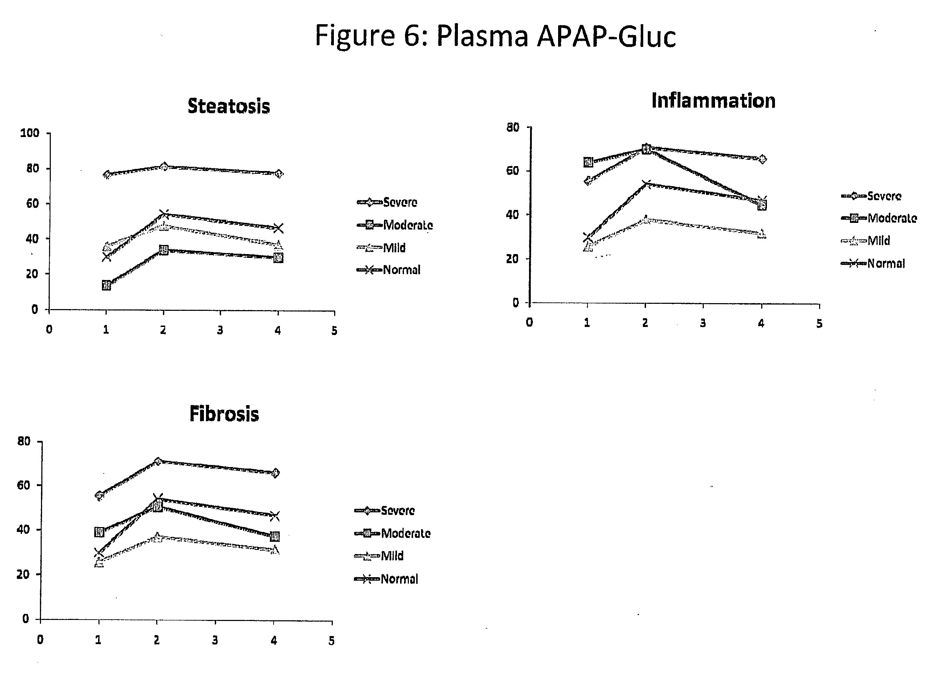

HPLC measurements and other experimental details are as described in Example 3; data are shown in Figure 6. Liver fibrosis was histologically characterized as mild (minimal scarring around blood vessels), moderate (scarring extending out from the liver blood vessels), or severe (scarring forming bridges between blood vessels).

### Example 5. Acetaminophen-Glucuronide levels are higher in the plasma of pediatric patients with hepatic inflammation than in patients without hepatic inflammation

HPLC measurements and other experimental details are as described in Example 3; data are shown in Figure 6. Liver inflammation was histologically characterized as mild (less than 2 foci of inflammation), moderate (2-4 foci of inflammation), or severe (more than 4 foci of inflammation).

## Claims

1. A method for diagnosing non-alcoholic steatohepatitis (NASH) or hepatic fibrosis in a human, comprising:
(a) determining an amount of acetaminophen (APAP)-glucuronide in a plasma sample and/or a urine sample obtained from a human subject after the human subject was administered acetaminophen (APAP), wherein the human subject is at risk of non-alcoholic steatohepatitis (NASH) or hepatic fibrosis;
(b) comparing the amount of APAP-glucuronide in one or both of the plasma sample and the urine sample to a control; and
(c) identifying the human subject as having non-alcoholic steatohepatitis (NASH) or hepatic fibrosis if the amount of APAP-glucuronide in one or both of the plasma sample and the urine sample is elevated relative to the control.

2. The method of claim 1, wherein the human subject at risk of NASH suffers from one or more of non-alcoholic fatty liver disease; metabolic syndrome, obesity, dyslipidemia, insulin resistance, and diabetes.

3. The method of claim 1, wherein the human subject at risk of hepatic fibrosis suffers from, or previously suffered from, one or more conditions selected from the group consisting of α₁-antitrypsin deficiency, Wilson's disease, fructosemia, galactosemia, Type III, IV, VI, IX, and X glycogen storage diseases, hemochromatosis, Gaucher's disease, Zellweger syndrome, tyrosinemia, bacterial infection, viral infection, parasitic infection, Budd-Chiari syndrome, alcoholism, drug addiction, heart failure, hepatic veno-occlusive disease, and portal vein thrombosis.

4. The method of any one of claims 1-3, wherein the amount of APAP-glucuronide is measured in a plasma sample, and wherein a measurement is made between 1 minute and 180 minutes after APAP administration, or wherein the amount of APAP-glucuronide is measured in a urine sample, and wherein a measurement is made between 120 minutes and 480 minutes after APAP administration.

5. A method for identifying a human subject at risk of an adverse drug reaction, comprising
(a) determining an amount of acetaminophen (APAP)-glucuronide in a plasma sample and/or a urine sample obtained from a human subject after the human subject was administered acetaminophen (APAP), wherein the human subject is in need of treatment with a therapeutic drug selected from the group consisting of doxorubicin, morphine, cisplatin, ectoposide, methotrexate, glucuronidated conjugated drugs, glutathione-conjugated drugs, and sulfate-conjugated drugs;
(b) comparing the amount of APAP-glucuronide in one or both of the plasma sample and the urine sample to a control; and
(c) identifying the human subject as at risk of an adverse drug reaction if the amount of APAP-glucuronide in one or both of the plasma sample and the urine sample is elevated relative to the control.

6. A method for determining an appropriate dosage of a therapeutic drug selected from the group consisting of doxorubicin, morphine, cisplatin, ectoposide, methotrexate, glucuronidated conjugated drugs, glutathione-conjugated drugs, and sulfate-conjugated drugs for a human subject, comprising
(a) determining an amount of acetaminophen (APAP)-glucuronide in a plasma sample and/or a urine sample obtained from a human subject after the human subject was administered acetaminophen (APAP), wherein the human subject is in need of treatment with a therapeutic drug selected from the group consisting of doxorubicin, morphine, cisplatin, ectoposide, methotrexate, glucuronidated conjugated drugs, glutathione-conjugated drugs, and sulfate-conjugated drugs;
(b) comparing the amount of APAP-glucuronide in one or both of the plasma sample and the urine sample to a control; and
(c) determining that the human subject should receive a non-standard dosage of the drug if the amount of APAP-glucuronide in one or both of the plasma sample and the urine sample is elevated relative to the control.

7. The method of any one of the preceding claims, wherein the amount of APAP-glucuronide is measured in a plasma sample, and wherein a measurement is made between 1 minute and 180 minutes after APAP administration.

8. The method of any one of the preceding claims, wherein the amount of APAP-glucuronide is measured in a urine sample, and wherein a measurement is made between 120 minutes and 480 minutes after APAP administration.

9. The method of any one of the preceding claims, wherein between 250 mg and 1000 mg of APAP was administered to the human subject.

10. The method of any one of the preceding claims, wherein the determining, comparing, and identifying steps are automated.

11. A machine readable storage media, comprising a set of instructions for causing a metabolite measuring device to carry out the determining, comparing, and identifying steps of any one of the preceding claims.

## Patentansprüche

1. Verfahren zum Diagnostizieren von nicht-alkoholischer Steatohepatitis (NASH) oder hepatischer Fibrose in einem Menschen, welches umfasst:
(a) Bestimmen einer Menge an Acetaminophen (APAP)-Glucuronid in einer Plasmaprobe und/oder einer Urinprobe, erhalten von einer menschlichen Person, nachdem der menschlichen Person Acetaminophen (APAP) verabreicht wurde, wobei die menschliche Person ein Risiko für nicht-alkoholische Steatohepatitis (NASH) oder hepatische Fibrose hat;
(b) Vergleichen der Menge an APAP-Glucuronid in einer oder beiden der Plasmaprobe und der Urinprobe mit einer Kontrolle; und
(c) Identifizieren, dass die menschliche Person nicht-alkoholische Steatohepatitis (NASH) oder hepatische Fibrose hat, wenn die Menge an APAP-Glucuronid in einer oder beiden der Plasmaprobe und der Urinprobe bezogen auf die Kontrolle erhöht ist.

2. Verfahren nach Anspruch 1, wobei die menschliche Person mit einem Risiko für NASH an einem oder mehreren von nicht-alkoholischer Fettlebererkrankung; metabolischem Syndrom, Adipositas, Dyslipidämie, Insulinresistenz und Diabetes leidet.

3. Verfahren nach Anspruch 1, wobei die menschliche Person mit einem Risiko für hepatische Fibrose an einem oder mehreren Zuständen leidet oder vorangehend gelitten hat, ausgewählt aus der Gruppe bestehend aus α₁-Antitrypsin-Mangel, Morbus Wilson, Fructosämie, Galaktosämie, Typ III, IV, VI, IX und X Glykogenspeicherkrankheiten, Hämochromatose, Morbus Gaucher, Zellweger-Syndrom, Tyrosinämie, bakterieller Infektion, Virusinfektion, parasitärer Infektion, Budd-Chiari-Syndrom, Alkoholismus, Drogenabhängigkeit, Herzversagen, hepatischer Venenverschlusskrankheit und Pfortaderthrombose.

4. Verfahren nach einem von Ansprüchen 1-3, wobei die Menge an APAP-Glucuronid in einer Plasmaprobe gemessen wird und wobei eine Messung zwischen 1 Minute und 180 Minuten nach APAP-Verabreichung durchgeführt wird, oder wobei die Menge an APAP-Glucuronid in einer Urinprobe gemessen wird und wobei eine Messung zwischen 120 Minuten und 480 Minuten nach APAP-Verabreichung durchgeführt wird.

5. Verfahren zum Identifizieren einer menschlichen Person mit einem Risiko für eine Arzneimittelnebenwirkung, welches umfasst:
(a) Bestimmen einer Menge an Acetaminophen (APAP)-Glucuronid in einer Plasmaprobe und/oder einer Urinprobe, erhalten von einer menschlichen Person, nachdem der menschlichen Person Acetaminophen (APAP) verabreicht wurde, wobei die menschliche Person eine Behandlung mit einer therapeutischen Arznei benötigt, ausgewählt aus der Gruppe bestehend aus Doxorubicin, Morphin, Cisplatin, Ectoposid, Methotrexat, glucuronidierten konjugierten Arzneien, Glutathion-konjugierten Arzneien und Sulfat-konjugierten Arzneien;
(b) Vergleichen der Menge an APAP-Glucuronid in einer oder beiden der Plasmaprobe und der Urinprobe mit einer Kontrolle; und
(c) Identifizieren, dass die menschliche Person ein Risiko für eine Arzneimittelnebenwirkung hat, wenn die Menge an APAP-Glucuronid in einer oder beiden der Plasmaprobe und der Urinprobe bezogen auf die Kontrolle erhöht ist.

6. Verfahren zum Bestimmen einer passenden Dosierung einer therapeutischen Arznei, ausgewählt aus der Gruppe bestehend aus Doxorubicin, Morphin, Cisplatin, Ectoposid, Methotrexat, glucuronidierten konjugierten Arzneien, Glutathion-konjugierten Arzneien und Sulfat-konjugierten Arzneien, für eine menschliche Person, welches umfasst:
(a) Bestimmen einer Menge an Acetaminophen (APAP)-Glucuronid in einer Plasmaprobe und/oder einer Urinprobe, erhalten von einer menschlichen Person, nachdem der menschlichen Person Acetaminophen (APAP) verabreicht wurde, wobei die menschliche Person Behandlung mit einer therapeutischen Arznei benötigt, ausgewählt aus der Gruppe bestehend aus Doxorubicin, Morphin, Cisplatin, Ectoposid, Methotrexat, glucuronidierten konjugierten Arzneien, Glutathion-konjugierten Arzneien und Sulfat-konjugierten Arzneien;
(b) Vergleichen der Menge an APAP-Glucuronid in einer oder beiden der Plasmaprobe und der Urinprobe mit einer Kontrolle; und
(c) Bestimmen, dass die menschliche Person eine Nicht-Standarddosierung der Arznei erhalten sollte, wenn die Menge an APAP-Glucuronid in einer oder beiden der Plasmaprobe und der Urinprobe bezogen auf die Kontrolle erhöht ist.

7. Verfahren nach einem der vorangehenden Ansprüche, wobei die Menge an APAP-Glucuronid in einer Plasmaprobe gemessen wird und wobei eine Messung zwischen 1 Minute und 180 Minuten nach APAP-Verabreichung durchgeführt wird.

8. Verfahren nach einem der vorangehenden Ansprüche, wobei die Menge an APAP-Glucuronid in einer Urinprobe gemessen wird, und wobei eine Messung zwischen 120 Minuten und 480 Minuten nach APAP-Verabreichung durchgeführt wird.

9. Verfahren nach einem der vorangehenden Ansprüche, wobei zwischen 250 mg und 1000 mg APAP an die menschliche Person verabreicht wurden.

10. Verfahren nach einem der vorangehenden Ansprüche, wobei die Schritte des Bestimmens, Vergleichens und Identifizierens automatisiert sind.

11. Maschinenlesbare Speichermedien, welche einen Satz Anweisungen umfassen, um eine Metabolit-Messvorrichtung zu veranlassen, die Schritte des Bestimmens, Vergleichens und Identifizierens nach einem der vorangehenden Ansprüche durchzuführen.

## Revendications

1. Procédé de diagnostic de la stéatohépatite non alcoolique (NASH) ou de la fibrose hépatique chez un être humain, comprenant :
(a) la détermination d'une quantité d'acétaminophène (APAP)-glucuronide dans un échantillon de plasma et/ou un échantillon d'urine obtenu d'un sujet humain après que l'on a administré de l'acétaminophène (APAP) au sujet humain, le sujet humain présentant un risque de souffrir d'une stéatohépatite non alcoolique (NASH) ou d'une fibrose hépatique ;
(b) la comparaison de la quantité d'APAP-glucuronide dans l'un ou les deux échantillon(s) parmi l'échantillon de plasma et l'échantillon d'urine à un témoin ; et
(c) l'identification du sujet humain en tant que sujet ayant une stéatohépatite non alcoolique (NASH) ou une fibrose hépatique si la quantité d'APAP-glucuronide dans l'un ou les deux échantillon(s) parmi l'échantillon de plasma et l'échantillon d'urine est élevée par rapport au témoin.

2. Procédé selon la revendication 1, dans lequel le sujet humain risquant de présenter une NASH souffre d'une ou plusieurs stéatose(s) hépatique(s) non alcoolique(s) ; d'un syndrome métabolique, d'une obésité, d'une dyslipidémie, d'une insulino-résistance et d'un diabète.

3. Procédé selon la revendication 1, dans lequel le sujet humain risquant de présenter une fibrose hépatique souffre ou a précédemment souffert d'une ou plusieurs affections choisies dans le groupe comprenant un déficit en α₁-antitrypsine, la maladie de Wilson, une fructosémie, une galactosémie, des maladies du stockage du glycogène de type III, IV, VI, IX et X, une hémochromatose, la maladie de Gaucher, le syndrome de Zellweger, une tyrosinémie, une infection bactérienne, une infection virale, une infection parasitaire, le syndrome de Budd-Chiari, l'alcoolisme, une toxicomanie, une insuffisance cardiaque, une maladie véno-occlusive hépatique et une thrombose de la veine porte.

4. Procédé selon l'une quelconque des revendications 1 à 3, dans lequel la quantité d'APAP-glucuronide est mesurée dans un échantillon de plasma, et dans lequel une mesure est réalisée entre 1 minute et 180 minutes après l'administration d'APAP ou dans lequel la quantité d'APAP-glucuronide est mesurée dans un échantillon d'urine, et dans lequel une mesure est réalisée entre 120 minutes et 480 minutes après l'administration d'APAP.

5. Procédé d'identification d'un sujet humain risquant de présenter une réaction médicamenteuse indésirable, comprenant :
(a) la détermination d'une quantité d'acétaminophène (APAP)-glucuronide dans un échantillon de plasma et/ou un échantillon d'urine obtenu d'un sujet humain après que l'on a administré de l'acétaminophène (APAP) au sujet humain, le sujet humain ayant besoin d'un traitement avec un médicament thérapeutique choisi dans le groupe comprenant la doxorubicine, la morphine, le cisplatine, l'ectoposide, le méthotrexate, les médicaments de conjugué glucuronidé, les médicaments conjugués au glutathion et les médicaments de conjugué sulfate ;
(b) la comparaison de la quantité d'APAP-glucuronide dans l'un ou les deux échantillon(s) parmi l'échantillon de plasma et l'échantillon d'urine à un témoin ; et
(c) l'identification du sujet humain en tant que sujet risquant de présenter une réaction médicamenteuse indésirable si la quantité d'APAP-glucuronide dans l'un ou les deux échantillon(s) parmi l'échantillon de plasma et l'échantillon d'urine est élevée par rapport au témoin.

6. Procédé de détermination d'un dosage approprié d'un médicament thérapeutique choisi dans le groupe comprenant la doxorubicine, la morphine, le cisplatine, l'ectoposide, le méthotrexate, les médicaments de conjugué glucuronidé, les médicaments conjugués au glutathion et les médicaments de conjugué sulfate pour un sujet humain, comprenant :
(a) la détermination d'une quantité d'acétaminophène (APAP)-glucuronide dans un échantillon de plasma et/ou un échantillon d'urine obtenu d'un sujet humain après que l'on a administré de l'acétaminophène (APAP) au sujet humain, le sujet humain ayant besoin d'un traitement avec un médicament thérapeutique choisi dans le groupe comprenant la doxorubicine, la morphine, le cisplatine, l'ectoposide, le méthotrexate, les médicaments de conjugué glucuronidé, les médicaments conjugués au glutathion et les médicaments de conjugué sulfate ;
(b) la comparaison de la quantité d'APAP-glucuronide dans l'un ou les deux échantillon(s) parmi l'échantillon de plasma et l'échantillon d'urine à un témoin ; et
(c) la détermination du fait que le sujet humain devrait recevoir un dosage non standard du médicament si la quantité d'APAP-glucuronide dans l'un ou les deux échantillon(s) parmi l'échantillon de plasma et l'échantillon d'urine est élevée par rapport au témoin.

7. Procédé selon l'une quelconque des revendications précédentes, dans lequel la quantité d'APAP-glucuronide est mesurée dans un échantillon de plasma, et dans lequel une mesure est réalisée entre 1 minute et 180 minutes après administration de l'APAP.

8. Procédé selon l'une quelconque des revendications précédentes, dans lequel la quantité d'APAP-glucuronide est mesurée dans un échantillon d'urine, et dans lequel une mesure est réalisée entre 120 minutes et 480 minutes après l'administration d'APAP.

9. Procédé selon l'une quelconque des revendications précédentes, dans lequel entre 250 mg et 1000 mg d'APAP sont administrés au sujet humain.

10. Procédé selon l'une quelconque des revendications précédentes, dans lequel les étapes de détermination, de comparaison et d'identification sont automatisées.

11. Support de stockage pouvant être lu par une machine, comprenant un ensemble d'instructions pour qu'un dispositif de mesure d'un métabolite réalise les étapes de détermination, de comparaison et d'identification selon l'une quelconque des revendications précédentes.
